(19)

Europäisches
Patentamt

European
Patent Office

Office européen
des brevets

(11) **EP 2 016 405 B1**

(12) **EUROPEAN PATENT SPECIFICATION**

| | |
|---|---|
| (45) Date of publication and mention<br>of the grant of the patent:<br>**27.09.2017 Bulletin 2017/39** | (51) Int Cl.:<br>***G06F 19/24*** (2011.01)    ***G01N 33/574*** (2006.01)<br>*G06F 19/20* (2011.01) |
| (21) Application number: **07761281.0** | (86) International application number:<br>**PCT/US2007/067418** |
| (22) Date of filing: **25.04.2007** | (87) International publication number:<br>**WO 2007/130831 (15.11.2007 Gazette 2007/46)** |

(54) **METHODS AND APPARATUS FOR IDENTIFYING DISEASE STATUS USING BIOMARKERS**

VERFAHREN UND VORRICHTUNG ZUR ERKENNUNG EINES KRANKHEITSZUSTANDES
ANHAND VON BIOMARKERN

PROCÉDÉS ET DISPOSITIFS POUR IDENTIFIER UN ÉTAT PATHOLOGIQUE EN UTILISANT DES
BIOMARQUEURS

| | |
|---|---|
| (84) Designated Contracting States:<br>**AT BE BG CH CY CZ DE DK EE ES FI FR GB GR<br>HU IE IS IT LI LT LU LV MC MT NL PL PT RO SE<br>SI SK TR** | (56) References cited:<br>**US-A1- 2002 006 616    US-A1- 2005 176 057<br>US-B1- 6 714 925    US-B2- 6 949 342** |
| (30) Priority: **01.05.2006  US 381104** | • **GOLUB T R ET AL: "Molecular classification of<br>cancer: Class discovery and class prediction by<br>gene expression monitoring", SCIENCE,<br>AMERICAN ASSOCIATION FOR THE<br>ADVANCEMENT OF SCIENCE, WASHINGTON,<br>DC; US, vol. 286, no. 5439, 15 October 1999<br>(1999-10-15), pages 531-537, XP002207658, ISSN:<br>0036-8075, DOI: 10.1126/SCIENCE.286.5439.531** |
| (43) Date of publication of application:<br>**21.01.2009  Bulletin 2009/04** | |
| (73) Proprietor: **Provista Diagnostics, LLC<br>Phoenix AZ 85016 (US)** | • **CLIP: 'A new prognostic system for<br>hepatocellular carcinoma: a retrospective study<br>of 435 patients' HEPATOLOGY vol. 28, 1998,<br>pages 751 - 755, XP008131674** |
| (72) Inventor: **RANDALL, Grimes, F.<br>Scottsdale, AZ 85254 (US)** | |
| (74) Representative: **Bates, Philip Ian<br>Reddie & Grose LLP<br>The White Chapel Building<br>10 Whitechapel High Street<br>London E1 8QS (GB)** | |

**Description**

FIELD OF THE INVENTION

The invention relates generally to methods and apparatus for identifying disease status in a patient, and more particularly to identifying disease status in a patient according to levels of one or more biomarkers.

BACKGROUND OF THE INVENTION

Biomarkers are used in medicine to help diagnose or determine the presence, absence, status and/or stage of particular diseases, Diagnostically useful biomarkers have been identified using measured levels of a single biomarker obtained from a statistically significant number of disease-negative and disease-positive subjects in a population and establishing a mean and a standard deviation tor the disease negative and positive stales. If the measured biomarker concentrations for the disease-positive and -negative states were found to have widely separated Gaussian or nearly Gaussian distributions, the biomarker was considered useful for predicting instances of the disease. Subsequent patients could be considered disease-positive if the patient's biomarker concentration was above (or, in some cases, below) a cut point generally defined as a biomarker concentration that is between the disease-positive and disease-negative means and two to three standard deviations away from the disease slate negative mean.

While conventional methods have produced clinically useful biomarkers, their application to determining a variety of disease statuses in subjects is limited for at least five reasons. First, these methods presume a normal, Gaussian data distribution in the population, where all measured biomarker concentrations are roughly distributed symmetrically above and below a mean and take the shape of a bell curve. In such cases, approximately 68% of the data is within one standard deviation of the mean, 95% of the data is within two standard deviations of the mean, and 99.7% of the data is within three standard deviations of the mean in either the disease-positive or -negative cohort. This assumption, however, only holds true for a fraction of all potential biomarkers. Human biochemistry is a complex system in which many components serve multiple functions and are themselves regulated by a variety of other components. As such, it is common to find biomarkers that display non-Gaussian distributions, which include values that lie substantially apart (at the far high end and/or far low end of the distribution) from the bulk of the values, and may span several orders of magnitude.

Second, traditional methods rely on the analysis of a single biomarker to indicate a disease state. Given the complex interaction of human biochemistry, however, the interaction of multiple markers often have a bearing on the presence or absence of disease. Instead of integrating multiple statistically significant markers, single marker models rely on the ideal (or nearly ideal) performance of a single marker, which may result in a less accurate diagnosis of a disease state than integrating multiple biomarkers.

Third, conventional methods rely exclusively on large differences between disease-negative and disease-positive populations, and disregard all information when the distributions of the disease-negative and disease-positive populations overlap to any significant degree. In traditional single marker models, differences between the means of the negative disease stale and the positive disease state that are less than one and one-half to two standard deviations ore considered to have little or no value, even when these differences are found to be persistent and reproducible.

Fourth, the traditional single marker methods are often confounded by biodiversity and the presence of sub-groups in the disease-negative or disease-positive populations. Given the complexity of human biochemistry, many factors can affect the measured concentration of a given biomarker, such as a patient's demographic characteristics, family history and medical history. All of these factors may increase the potential marker's observed variability and standard deviation, masking or obscuring the relationship to the disease state.

Finally, despite increasing understanding of biomarkers and availability of convenient biomarker assays (e.g., immunohistochemistry assays) to detect and quantify expression of specific biomarkers associated with a disease, traditional analyses often fail to sufficiently differentiate the disease-negative and disease-positive statuses to permit reliable diagnosis of diseases.

US6714925 relates to a system for identifying patterns in biological data using a distributed network.

The following document relates to a method of classifying cancer based on gene expression monitoring by DNA microarrays:

Golub T R et al., "Molecular classification of cancer: Class discovery and class prediction by gene expression monitoring", Science, American Association for the Advancement of Science, vol. 286, no. 5439, pages 531 - 537.

US2005176057 relates to methods of diagnosis, evaluation, and treatment of mood disorders, particularly bipolar disorder.

US6949342 relates to methods of identifying prostate cancer and outcome prediction by expression analysis.

SUMMARY OF THE INVENTION

According to an aspect of the present invention, there is provided a method for assessing a breast cancer disease status of a patient, comprising:

obtaining a first data set for a plurality of biomarkers in a condition-positive cohort;
obtaining a second data set for the plurality of biomarkers in a condition-negative cohort; wherein the plurality of biomarkers comprises prostate-specific antigen (PSA), tumor necrosis factor alpha (TNF-$\alpha$), interleukin-6 (IL-6), interleukin-8 (IL-8), vascular endothelial growth factor (VEGF), and riboflavin carrier protein (RCP);
processing the first data set and the second data set to reduce the influence of non-normal biomarker levels with a non-Gaussian distribution within the condition-positive cohort and the condition-negative cohort by assigning maximum and/or minimum allowable values for each biomarker to produce a first processed data set and a second processed data set;
generating a disease status model by selection of at least one informative biomarker from the first processed data set as compared to the second processed data set using an analysis configured to remove a biomarker that is uninformative of disease status;
inputting a patient data set for the at least one informative biomarker into the disease status model; and
determining the breast cancer disease status of the patient.

According to a further aspect of the present invention, there is provided a system for assessing a breast cancer disease status of a patient, comprising a computer system configured to:

receive a first data set for a plurality of biomarkers in a condition-positive cohort;
receive a second data set for a plurality of biomarkers in a condition-negative cohort; wherein the plurality of biomarkers comprises prostate-specific antigen (PSA), tumor necrosis factor alpha (TNF-$\alpha$), interleukin-6 (IL-6), interleukin-8 (IL-8), vascular endothelial growth factor (VEGF), and riboflavin carrier protein (RCP);_process the first data set and the second data set to reduce the influence of non-normal biomarker levels with a non-Gaussian distribution within the condition-positive cohort and the condition-negative cohort by assigning maximum and/or minimum allowable values for each biomarker to produce a first processed data set and a second processed data set; and
generate a disease status model by selection of at least one informative biomarker from the first processed data set as compared to the second processed data set using an analysis configured to remove a biomarker that is uninformative of disease status;
inputting a patient data set for the at least one informative biomarker into the disease status model; and
determining the breast cancer disease status of the patient.

In a further aspect, there is provided a computer program configured to cause a computer to execute a method for assessing a disease status of a human according to the method set out above.

BRIEF DESCRIPTION OF THE DRAWING FIGURES

A more complete understanding of the present invention may be derived by referring to the detailed description when considered in connection with the following illustrative figures. In the following figures, like reference numbers refer to similar elements and steps.

Figure 1 is a block diagram of a computer system.
Figure 2 is a flow chart of a process for identifying disease status.
Figure 3 is a flow chart of a process for controlling a range of values.
Figure 4 is a flow chart of a process for normalizing data.
Figure 5 is a flow chart of a process for classifying data according to cut points.
Figure 6 is a plot of cumulative frequencies of disease-positive and disease-negative biomarker concentrations.
Figure 7 is a flow chart of a process for establishing a disease status model.
Figure 8 is a flow chart of a process for identifying disease status in an individual.
Figure 9 is a plot of cumulative frequencies of breast cancer positive and breast cancer negative concentrations versus PSA concentration.
Figure 10 illustrates data scoring model for selecting one or more cut points.

Elements and steps in the figures are illustrated for simplicity and clarity and have not necessarily been rendered according to any particular sequence. For example, steps that may be performed concurrently or in different order are illustrated in the figures to help to improve understanding of embodiments of the present invention.

DETAILED DESCRIPTION OF THE INVENTION

The present invention is described partly in terms of functional components and various processing steps. Such functional components and processing steps may be realized by any number of components, operations and techniques configured to perform the specified functions and achieve the various results. For example, the present invention may employ various biological samples, biomarkers, elements, materials, computers, data sources, storage systems and media, information gathering techniques and processes, data processing criteria, statistical analyses, regression analyses and the like, which may carry out a variety of functions.

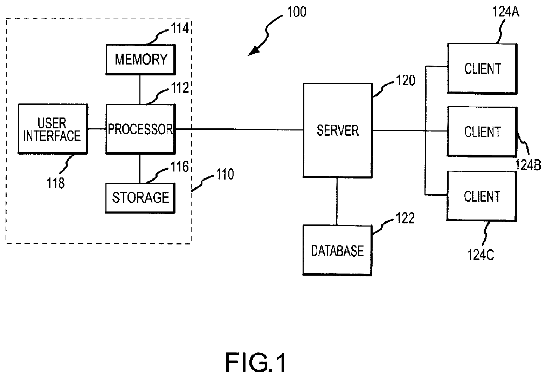

Methods and systems for analyzing biomarker information according to various aspects of the present invention may be implemented in any suitable manner, for example using a computer program operating on the computer system. Referring to Figure 1, an exemplary biomarker analysis system 100 according to various aspects of the present invention may be implemented in conjunction with a computer system 110, for example a conventional computer system comprising a processor 112 and a random access memory 114, such as a remotely-accessible application server, network server, personal computer or workstation. The computer system 110 also suitably includes additional memory devices or information storage systems, such as a mass storage system 116 and a user interface 118, for example a conventional monitor, keyboard and tracking device. The computer system 110 may, however, comprise any suitable computer system and associated equipment and may be configured in any suitable manner. In one embodiment, the computer system 110 comprises a stand-alone system. In another embodiment, the computer system 110 is part of a network of computers including a server 120 and a database 122. The database stores information that may be made accessible to multiple users 124A-C, such as different users connected to the server 120. In the present embodiment, the server 120 comprises a remotely-accessible server, such as an application server that may be accessed via a network, such as a local area network or the Internet.

The software required for receiving, processing, and analyzing biomarker information may be implemented in a single device or implemented in a plurality of devices. The software may be accessible via a network such that storage and processing of information takes place remotely with respect to users 124A-C. The biomarker analysis system 100 provide functions and operations to facilitate biomarker analysis, such as data gathering, processing, analysis, reporting and/or diagnosis. The present biomarker analysis system 100 maintains information relating to biomarkers and facilitates the analysis and/or diagnosis. For example, the computer system 110 executes the computer program, which may receive, store, search, analyze, and report information relating to biomarkers. The computer program may comprise multiple modules performing various functions or operations, such as a processing module for processing raw data and generating supplemental data and an analysis module for analyzing raw data and supplemental data to generate a disease status model and/or diagnosis information.

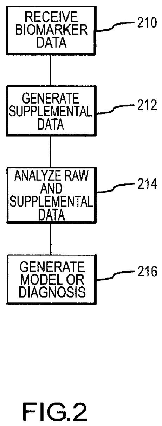

The procedures performed by the biomarker analysis system 100 may comprise any suitable processes to facilitate biomarker analysis and/or diagnosis. The biomarker analysis system 100 may be configured to establish a disease status model and/or determine disease status in a patient. Determining or identifying disease status may comprise generating any useful information regarding the condition of the patient relative to the disease, such as performing a diagnosis, providing information helpful to a diagnosis, assessing the stage or progress of a disease, identifying a condition that may indicate a susceptibility to the disease, identify whether further tests may be recommended, or otherwise assess the disease status, likelihood of disease, or other health aspect of the patient. Referring to Figure 2, the biomarker analysis system 100 may receive raw biomarker data and subject data (210) relating to one or more individuals providing the biological samples from which the biomarker data is drawn. The biomarker analysis system 100 processes the raw data and subject data to generate supplemental data (212), and analyzes the raw data, subject data, and/or supplemental data (214) to establish a disease state model and/or a patient diagnosis (216).

The biomarker analysis system 100 may also provide various additional modules and/or individual functions. For example, the biomarker analysis system 100 may also include a reporting function, for example to provide information relating to the processing and analysis functions. The biomarker analysis system 100 may also provide various administrative and management functions, such as controlling access and performing other administrative functions.

The biomarker analysis system 100 suitably generates a disease status model and/or provides a diagnosis for a patient based on raw biomarker data and/or additional subject data relating to the subjects in the cohorts. The biomarker data may be acquired from any suitable biological samples containing measurable amounts of the biomarkers.

The biomarker data may be obtained and processed to establish a disease status model that incorporates data from a plurality of biomarkers, such as data from members of disease-negative and disease-positive cohorts or other condition-positive and/or -negative groups. The biological samples are suitably obtained from a statistically significant number of disease-positive and -negative subjects. Disease-positive and -negative cohorts may contain a sufficient number of subjects to ensure that the data obtained are substantially characteristic of the disease-negative and disease-positive states, such as statistically representative groups. For example, each cohort may have at least 30 subjects in each cohort. Each cohort may be characterized by several sub-cohorts, reflecting, for example, that the disease can exist in disease-positive individuals at various stages, or other demographic, behavioral, or other factors that may affect the biomarker levels in either disease-positive or -negative individuals.

The biomarker analysis system 100 may utilize any single or combination of biological materials from which the levels of potential biomarkers may be reproducibly determined. Levels of all measured biomarkers may be obtained from as few sample sources as possible, such as from a single, readily obtained sample. For example, sample sources may include, but are not limited to, whole blood, serum, plasma, urine, saliva, mucous, aspirates (including nipple aspirates) or tissues (including breast tissue or other tissue sample). Biomarker levels may vary from source-to-source and disease-indicating levels may be found only in a particular sample source. Consequently, the same sample sources are suitably used both for creating disease status models and evaluating patients. If a disease status model is constructed from biomarker levels measured in whole blood, then the test sample from a patient may also be whole blood. Where samples are processed before testing, all samples may be treated in a like manner and randomly collected and processed.

The biomarker analysis system 100 may analyze any appropriate quantity or characteristic. In the present case, a biomarker may comprise any disease-mediated physical trait that can be quantified, and may comprise a distinctive biochemical indicator of a biological process or event. Many biomarkers are available for use, and the biomarker analysis system 100 provides an analytical framework for modeling and evaluating biomarker level data.

Raw biomarker levels in the samples may be measured using any of a variety of methods, and a plurality of measuring tools may be used to acquire biomarker level data. For example, suitable measuring tools may include, but are not limited to, any suitable format of enzyme-linked immunosorbent assay (ELTSA), radioimmunoassay (RIA), flow cytometry, mass spectrometry or the like. As biomarker levels may vary from method to method and from procedure to procedure, the biomarker analysis system 100 may use consistent methods and procedures for creating disease status models as well as for evaluating patients. For example, if a disease status model is constructed from biomarker levels measured using a specified ELISA protocol, then the test sample from a patient should be measured using the same ELISA protocol.

The biomarker data, such as the raw biomarker levels and any other relevant data, are provided to the biomarker analysis system 100 for processing. One or more markers may be analyzed by the biomarker analysis system 100. The biomarker analysis system 100 may process the biomarker data to incorporate multiple markers, minimize potential impact of non-Gaussian distributions, and account for biodiversity. The biomarker analysis system 100 may analyse multiple biomarkers, assign boundary values for the biomarker levels, generate normalized data bused on the raw data and potentially relevant biomarker-affecting factors, compare biomarkers to cut points, and/or reduce the range of raw and/or adjusted data values. The biomarker analysis system 100 may also adjust the data for disease-specific risk factors and analyze the data to generate the disease status model.

The biomarker analysis system 100 may analyze multiple biomarkers to establish a disease status model and generate a diagnosis. Given the complex interaction of human biochemistry, multiple markers may have a relationship with the presence or absence of the disease state. Further, a single biomarker may not be associated exclusively with only one disease. While a single biomarker may provide useful information, diagnostic reliability may be improved by including a plurality of biomarkers, for example the most informative biomarkers. The biomarker analysis system 100 suitably integrates these multiple, less than ideal, but still statistically significant and informative biomarkers.

The biomarker analysis system 100 may assess whether a given biomarker is informative, such as according to a classification of not informative, informative, or highly informative, and whether it is productive to include the marker in the disease status model. For example, various biomarkers are associated with breast cancer and, when modeling characteristic biomarker levels and evaluating breast cancer in subjects, such markers may be highly relevant. In one particular example, up-regulated (elevated) and/or down-regulated (suppressed) levels in serum of prostate-specific antigen (PSA), tumor necrosis factor alpha (TNF-$\alpha$), interlcukin-6 (IL-6), interleukin-8 (IL-8), vascular endothelial growth factor (VEGF), and/or riboflavin carrier protein (RCP) are associated with breast cancer. Of these, RCP, TNF-$\alpha$, IL-8, and VEGF are more informative as to breast cancer status than the other two markers.

Human biochemistry is a complex system wherein many components serve multiple regulatory and other functions and are regulated by multiple other components. Often, biological data are non-Gaussian, particularly in a disease stale. As such, it is common to find biomarkers that display non-Gaussian distributions where measured values can include values that lie substantially apart from the bulk of the values, at the far high end, far low end, or both the high and low end of the distribution, and may span several orders of magnitude. The biomarker analysis system 100 may process the data to accommodate effects of nun-Gaussian distributions. Unlike Gaussian distributions, non-Gaussian distributions may be skewed to the left or to the right with respect to a data mean. Non-Gaussian distributions can be mathematically transformed to Gaussian distributions using logarithmic transformation. Non-Gaussian data can be subjected to sub-group averaging, data segmenting, using differential distributions, or using non-parametric statistics.

To integrate a plurality of biomarkers and control any adverse impact of non-Gaussian data points on the disease status model, the biomarker analysis system may pre-process the biomarker data to generate additional data to facilitate the analysis. For example, the biomarker analysis system 100 may impose various constraints upon, make adjustments to and/or calculate additional data from the raw biomarker level data to generate supplemental data comprising a set of variables in addition to the raw data that may be processed, for example using logistic regression to generate a linear model or other appropriate Statistical analysis that describes the relationship of the biomarkers to the disease state.

For example, the biomarker analysis system 100 may be configured to process the raw biomarker data to reduce negative effects of non-Gaussian distributions. In one embodiment, the biomarker analysis system 100 may reduce the influence of non-normal biomarker levels in biomarkers with non-Gaussian distributions, such as by assigning maximum and/or minimum allowable values or caps for each such biomarker. The caps may be assigned according to any suitable criteria, such as to encompass between about 66% and about 99.7% of the measured levels and exclude extraordinarily high values.

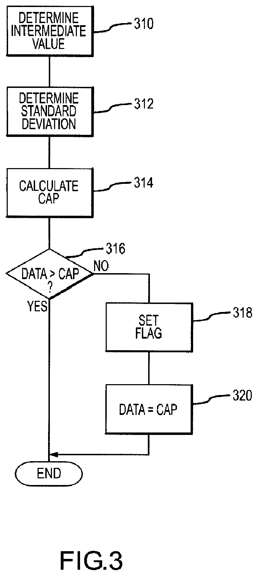

Referring to Figure 3, the maximum and/or minimum allowable values for each candidate biomarker may be established by first determining an intermediate value (310), such as the mean or median value, of that biomarker in the disease-negative cohort, and determining the standard deviation of n selected quantity of the measured biomarker levels (312), such as approximately 30% - 45% of the data points on either side of the median value when the data is plotted on a histogram, such that the central 60% to 90% of the measured data points are accounted for in determining the standard deviation. A maximum allowable value may be determined (314) according to the intermediate value and the standard deviation of the selected biomarker data, for example by adding to the median value to a multiple of the standard deviation, such as no more than four times the standard deviation, and more typically, an amount between one and a half and three times the standard deviation.

In the present embodiment, the biomarker analysis system 100 uses the median, instead of the mean, as the basis for determining the allowed maximum to more accurately reflect the majority of the values while reducing the impact of one or a few very high outlying, non-Gaussian values. Maximum values may also be calculated using data from any suitable set of data and any suitable technique or algorithm, such as data from a disease-positive cohort or from a mixture of disease-positive and disease-negative subjects. Maximum values may be calculated for each of the relevant biomarkers.

For example, in an embodiment of the present invention configured for detecting the presence of breast cancer, the maximum values for the applicable biomarkers are calculated by adding the median value of the biomarker for all subjects without breast cancer to two-and-a-half times the standard deviation of the marker for all subjects without breast cancer. In this exemplary embodiment, suitable median values for PSA, IL-6, TNF-$\alpha$. IL-8, and VEGF may be within ranges of 0.01 -10,0.5-25,0.1 -10, 5-150, and 100-5,000 picograms per milliliter (pg/ml) respectively, such as .53, .34,2.51, 52.12, and 329.98 pg/ml, respectively. Maximum values may he assigned for each of the biomarkers PSA, IL-6, TNF- $\alpha$, IL-8, and VEGF, for example within the ranges of 5-200, 10-300, 0.5-50, 100-2,000, and 500- 10,000 pg/ml, respectively, such as 122.15, 12.52, 48.01, 350.89, and 821.15 pg/ml, respectively. Thus, different maximum values may be calculated for the PSA, IL-6, RCP, TNF-$\alpha$, IL-8, and VEGF biomarkers, or for the RCP, TNF-$\alpha$, IL-8, and VEGF biomarkers

alone. In the present embodiment, these figures are determined using ELISA measurements for healthy women. The values may change as more data is added, variations in the ELISA procedure and/or test kits, reliance on data for disease-positive women, or use of non-ELISA techniques.

The resulting maximum allowable value may then be compared to the individual measured biomarker levels (316). If a particular subject's measured level is above the maximum value, a modification designator or flag, such as an integral value of 1 or 0 or other appropriate designator, may be associated with the subject's biomarker data, such as recorded in a particular field in his or her supplemental data set; if the biomarker level is below the maximum, an integral value of 0 is recorded in his or her supplemental data set (3 18). The designator criteria may be applied consistently between generating a disease status model and scoring an individual patient's biomarker levels to ease disease status model interpretation. The designators may also comprise more than just two discrete levels.

Additionally, when any of a subject's biomarker values exceed the maximum allowable value for that biomarker, the raw biomarker values may be replaced with the maximum allowable value for that biomarker (320). The adjusted data having capped values and additional designators may be part of the supplemental data, so that the raw data is preserved and the adjusted data with capped values and additional designators become part of the supplemental data set. The additional designator denotes that the measured values were unusually high, which may be informative about the disease status, while the replacement with the cap value limits the influence of the extremely high values. Without such caps, the extremely high values may "pull" the linear model to fit data that is the exception, not the norm.

Thus, if the patient's RCP biomarker exceeds the maximum allowable value, a flag is set in the subject's supplemental data to indicate that the RCP biomarker exceeded the limit and the raw biomarker level may be replaced with the maximum allowable value. Conversely, if the TNF-$\alpha$ biomarker level is within the range of accepted values, the original biomarker level is retained and the corresponding flag in the subject's supplemental data remains unset.

The biomarker analysis system 100 may also be configured to generate and analyze normalized data, for example based on the raw biomarker data and/or the capped supplemental data. Normalized data comprises the original data adjusted to account for variations observed in the measured values that may be attributed to one or more statistically significant biomarker level-affecting factors. For example, genetic, behavioral, age, medications, or other factors can increase or decrease the observed levels of specific biomarkers in an individual, independent of the presence or absence of a disease state. In the present embodiment, to detect breast cancer, potential factors that may substantially affect the levels of biomarkers indicative of breast cancer include: age; menopausal status; whether a hysterectomy has been performed; the usage of various hormones such birth control, estrogen replacement therapy. Tamoxifen or Raloxifene, and fertility drugs; the number or full-term pregnancies; the total number of months engaged in breast-feeding; prior breast biopsies; prior breast surgeries; a family history of breast cancer; height; weight; ethnicity; dietary habits; medicinal usage, including the use of NSAIDs; presence of other diseases; alcohol consumption; level of physical activity; and tobacco use.

Any suitable source or system may be used to identify factors that may affect a given biomarker, such as literature and research. In addition, any suitable processes or techniques may be used to determine whether particular factors are applicable and to what degree. For example, upon collecting the biological samples, members of the cohorts can be queried through subject questionnaires, additional clinical tests, or other suitable processes and mechanisms about various factors that can possibly affect the levels of their markers. The subject data containing this information relating to the subjects themselves may he provided to the biomarker analysis system 100 with the raw biomarker data, for example in the form of discrete and/or continuous variables.

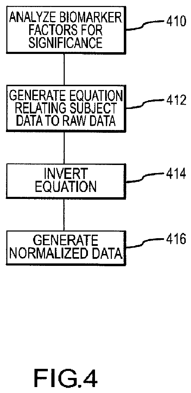

The relevance and effects of various factors upon biomarker levels may be assessed in any suitable manner. For example, when sample collection is completed, all biomarkers have been measured, and the raw data and subject data relating to the additional factors has been provided, the biomarker analysis system 100 may analyze the raw data and additional factors to identify such factors with a statistically significant affect. The biomarker analysis system 100 may also automatically select multiple relevant biomarkers from the plurality of biomarkers. Referring to Figure 4, the biomarker analysis system 100 may perform regression analyses or other appropriate statistical analyses using each biomarker as a dependent variable and the factors that potentially affect its level as independent variables (410). The biomarker analysis system 100 may, however, use any appropriate analysis to identify potential relationships between the factors and variations in the biomarker data.

Factors that are found to retain a p-value below a predetermined level (e.g., without limitation, $p<0.1$, $p<0.05$, or $p<0.025$) may be considered significant. The biomarker analysis system 100 may also be configured to compensate for the effects of such factors, such as by generating normalized data wherein the variation attributable to such factors has been removed from the analysis. For example, to remove factor-ascribed variation, raw data may be transformed using the inverse of a linear equation describing the relationship between the biomarker level and the factor or factors found to be significant. In one example of the present invention, the selected p-value to determine statistical significance for biomarkers specific to detecting breast cancer may be selected at .05. In another particular example, should linear regression or other appropriate analysis of raw data and subject show that a subject's age and gender affect a potential biomarker relating to Alzheimer's disease Y to a statistically significant level, the relationship the observed biomarker levels and the subject's age and gender could be described by the equation:

$$Y = M_1(Age) + M_2(Male) + B$$

where Y is the measured level of the potential Alzheimer's disease biomarker, $M_1$ and $M_2$ are the coefficients as determined by the linear regression, (Age) is a continuous variable that was found to be a statistically significant determinate of Y, (Male) is a discrete variable that was found to be a statistically significant determinate of Y, where 1 equals male and 0 equals female, and B is an intercept (412). To remove the variation in Y that can be ascribed to age and gender, a normalized or adjusted value Y' for the potential Alzheimer's disease biomarker Y may be calculated according to the inverse equation (414):

$$Y' = Y*(1/M_1)(Age) - M_2(Male)$$

Normalized data may be generated applying the inverse equation to the raw data and/or the supplemental data and added to the supplemental data. By removing variation due to known causes, a greater percentage of the remaining variation may be ascribed to the presence or absence of a disease state, thus clarifying a marker's relationship to the disease state that might otherwise be obscured. When statistically significant factors are identified as affecting the level of one or more potential biomarkers, both raw data and normalized data may be used in subsequent analyses. Analysis of normalized values may elucidate relationships that would otherwise be obscured, while raw data may provide greater ease of test administration and delivery.

The biomarker analysis system 100 may further process the raw and/or supplemental data in any suitable manner, such as to reduce the influence of non-Gaussian distributions. For example, the biomarker analysis system 100 may select one or more biomarker cut points and compare the raw and/or supplemental biomarker data to at least one designated biomarker cut point. Biomarker cut points may be selected according to any suitable criteria, such as according to known levels corresponding to disease or based on the raw and/or normalized biomarker data. For example, the biomarker analysis system 100 may compare cumulative frequency distributions of the condition-positive and -negative biomarker data for a particular biomarker and select one or more cut points for the biomarker according to a maximum difference between the condition-positive cumulative frequency distribution and the condition-negative cumulative frequency distribution for the selected biomarker.

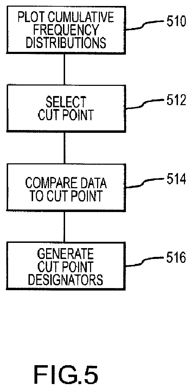

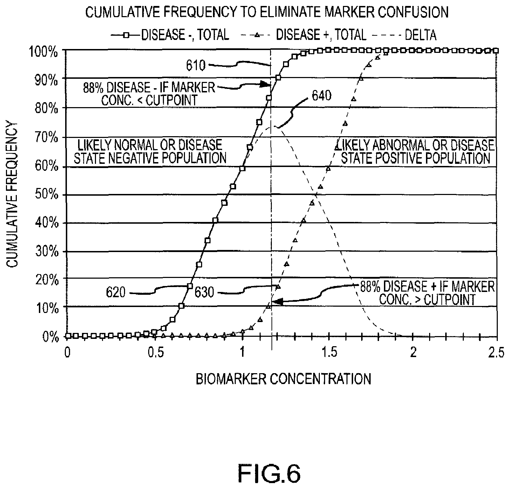

Referring to Figures 5 and 6, the biomarker analysis system 100 may designate at least one cut point for each biomarker. The biomarker analysis system 100 may initially generate cumulative frequency distributions for the raw and/or supplemental data for both the disease-positive cohort 630 and the disease-negative cohort 620 for each relevant biomarker (510), such as for each individual biomarker PSA, IL-6, RCP, TNF-$\alpha$, IL-8, and VEGF. The biomarker analysis system 100 may select one or more cut points (512), for example at a level where the difference between the cumulative frequency distribution of measured values in the disease-positive cohort and in the disease-negative cohort exceeds a predetermined value. The predetermined value may be any suitable threshold, such as where the cumulative frequency difference exceeds 10%, with higher values indicating greater difference between the positive and negative cohorts.

The present biomarker analysis system 100 may seek levels at which the difference between the positive and negative cohorts is greatest to establish cut points 640. A greater difference in the cumulative frequencies of the disease-positive and -negative states indicates a propensity to belong to either the disease-positive or disease-negative cohort. Conversely, potential markers that display less than a 10% difference in cumulative frequency at any point are less likely to be informative to a useful extent and may optionally be dropped from further analysis.

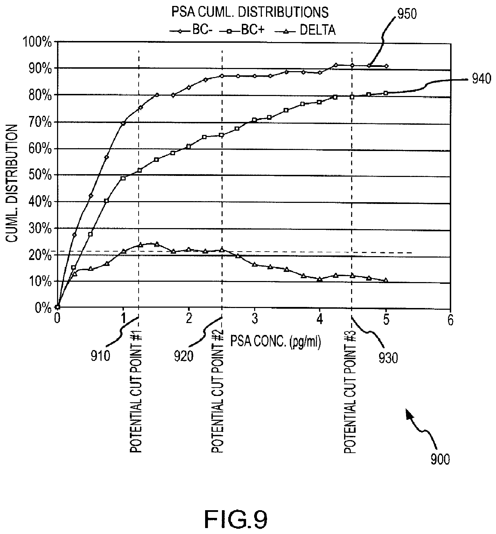

A cut point 640 may be selected even where the differences in cumulative frequency are low, particularly where the cut point may be deemed to be particularly informative, such as in the ease where there are no disease-positive or disease-negative values beyond a certain biomarker level. For example, referring to Figure 9, to detect breast cancer, cut-points for the biomarker PSA may be selected for values that are at a local maximum with an absolute difference exceeding 10% using a cumulative frequency plot 900. In this example, a first cut point 910 is selected at 1.25, a second cut point 920 is selected at 2.5, and a third cut point 930 is selected at 4.5. The differences in the cumulative frequency between disease-positive cohort plot 940 and disease-negative cohort plot 950 at each of the three cut points are 24%, 22%, and 12% respectively. The third cut point 930 may be suitably selected despite the relatively low difference in cumulative frequency since the lack of disease-negative values beyond a PSA concentration of 4.5 indicates a point that is particularly informative to the distribution.

Referring again to Figure 5, the raw and/or normalized biomarker data may be compared to the cut points (514) and the biomarker analysis system 100 may record a value indicating the result of the comparison as a cut point designator (516). The cut point designator may comprise any suitable value or indicator, such as the difference between the value and the cut point or other value. If a raw or normalized biomarker level is above the cut point, un integral value of 1 may be recorded as the cut point designator and stored in the supplemental data; if the level is below the cut point, an integral value of 0 may be recorded. The integral values could likewise indicate whether the biomarker levels are below the more than one cut-point, or exceed a cut point for some of a patient's biomarkers and not exceeding a cut point for others. Conversion of a continuous variable into a discrete variable indicates a propensity to belong to either a disease-positive or -negative cohort. All values on a particular side of a cut point may receive equal weight, regardless of how high or low they may be, which tends to eliminate the influence of non-Gaussian distributions.

The biomarker analysis system 100 may also be configured to reduce the range of values in data, for example where the range of measured or normalized level values for a biomarker is extremely wide. The range of values may be narrowed and the number of extremely high values reduced, while maintaining a meaningful distinction between values at the low and high ends of the range. The biomarker analysis system 100 may adjust the range of values in any suitable manner, for example by raising the measured values to fractional powers to obtain a set of reduced values for the biomarker. The biomarker analysis system 100 may select any suitable exponent values to maintain meaningful distinctions in the data. Meaningful distinctions can be lost if the range is narrowed too much by choosing a fractional power that is too small.

The biomarker analysis system may adjust the measured value for each biomarker, such as the PSA, IL-6, RCP, TNF-$\alpha$, IL-8, and VEGF

biomarkers, in each cohort member by raising each value to a fractional power. Multiple different fractional powers, such as exponential values ranging from ¾ to 1/10, such as 2/3 and 1/2, can be included in the analysis for each biomarker. Each reduced value may be Included in the supplemental data associated with the relevant biomarker's data set. The biomarker analysis system 100 may analyze the results, such as in the course of performing later regression analysis, to identity the fractional power value(s) that best accommodates the data, for example by removing those sets of values that lack statistical significance. Exponentially raising measured or normalized level values by fractional values reduces the data's range, allows linear models to better fit non-linear data, and provides a continuum of scoring where differing weights can be applied as high or low values. In an embodiment configured to detect breast cancer, for example, suitable fractional powers for the PSA, IL-6, RCP, TNF-$\alpha$, IL-8, and VEGF biomarkers may include 1/10, 1/5, 1/3, 1/2, and 2/3 for each of the relevant biomarkers.

The biomarker analysis system 100 may generate the disease status model on the raw data, the normalized data, any other supplemental data, and/or any additional disease risk factors that may have an impact or influence on specific risk for development of a disease. Given the complexity of human biochemistry, many factors can affect the measured concentration of one or more biomarkers, including, but not limited to, a patient's demographic characteristics, family history, and medical history. These factors all increase the potential markers' observed variabilities and standard deviations, masking or obscuring the relationship to the disease state.

The biomarker analysis system 100 may analyze and/or process disease risk factors that can affect a subject's risk, as well as biomarker factors that can affect biomarker levels differently as described above. The biomarker analysis system 100 may, for example, account for disease risk factors in the overall analysis of the data in conjunction with analyzing the marker specific scores. Considering risk factors accounts for differences in prevalence and essentially shifts the overall score to reflect the prevalence.

For example, as with the biomarker factors that can influence measured biomarker levels, disease risk factors may be included among the identified variables in determining the relationship between the variables and disease status. The additional disease risk factors may be selected according to any suitable criteria and/or from any suitable source. For example, technical literature may identify additional factors that have an impact or influence on specific risk for development of a particular disease of interest. Specific risk factors may include, without limitation, age, race, family history, date of menarche, menopausal status, depression, disease status, medication status, body mass index (BMI), date of first childbirth, head injuries, and/or other factors. When such disease risk factors arc known or suspected to be associated with a disease state, the subject's medical histories and/or the actual subject should be queried about the disease risk factors. This additional subject data may be provided to the biomarker analysis system 100, which may record the subjects' disease risk factor data with the subjects' biomarker (actor data as additional continuous or discrete variables.

The biomarker analysis system 100 suitably analyzes the data to identify relationships between the disease state and various raw data, supplemental data, and/or subject data. The relationship may be identified according to any suitable analysis and criteria. For example, the biomarker analysis system 100 may establish an equation, such as a linear equation, that describes a relationship between the identified variables and disease status. The biomarker analysis system 100 may apply any suitable analysis, such us one or more conventional regression analyses (e.g., linear regression, logistic regression, and/or Poisson regression) using the disease status as the dependent variable and one or more elements of the raw data and the supplemental data as the independent variables, or employ other analytical approaches, such as a generalized linear model approach, logit approach, discriminant function analysis, analysis of covariance, matrix algebra and calculus, and/or receiver operating characteristic approach. The biomarker analysis system 100 may automatically generate a statistical model for determining disease status according to differences between the biomarker data for the relevant biomarkers of the respective cohorts.

The present biomarker analysis system 100 may assess the relevance of a biomarker to a particular disease or condition according to any suitable technique or process. The biomarker analysis system 100 may perform statistical analyses of the biomarker data, such as statistical significance analyses. For example, the biomarker analysis system 100 may automatically generate a disease status model that eliminates non-informative and some less informative biomarkers, for example by disregarding all potential biomarkers that yield p-values less than a predetermined value upon statistical analysis against the disease status. The biomarker analysis system 100 may determine the relative contribution or strength of the remaining individual biomarkers, for example by the coefficients that the model applies to the markers or by the product of the coefficient of each marker and its range of values. Higher coefficients or products relative to those for other biomarkers in the model indicate more impact that the biomarker may be assigned for determining the disease state in the disease status model. The analysis may reduce the number of cut points and fractional exponent values used, in many cases to a single cut point and/or fractional exponent. Some of the factors are likely to relate to duplicate Information, so the biomarker analysis system 100 may select the factor that is most useful, such as the factor having the lowest p-value.

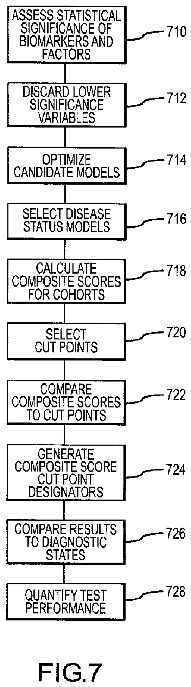

Referring to Figure 7, the biomarker analysis system 100 may perform an iterative analysis either starting with a single variable and adding variables one at a time, or starting with all variables and removing variables one at a time, until all variables are determined to be statistically significant, such as by having p-values lower than a predetermined level (e.g., without limitation, $p<0.1$, $p<0.05$, or $p<0.025$) (710). The iterative analysis may be configured to identify and remove biomarker data that is less informative regarding disease status than other data. For example, independent variables that demonstrate a p-value less than a predetermined value are retained in the model, while those with p-values higher than the predetermined value are discarded (712). The biomarker analysis system 100 may analyze multiple variations of additions and subtractions of variables to acquire an optimal solution (714), for example to maximize the model's adjusted R squared or the Bayesian information criterion and avoid sub-optimizing the model. For example, the resultant scoring model may take the form of the following equation:

$$y = m_1x_1 + m_2x_2 + m_3x_n + m_4d_1 + m_5d_2 + m_5d_n + b$$

where y is a continuous variable representing disease status;

$x_{1-n}$ are continuous variables, such as raw biomarker levels measured in biological samples and/or normalized or capped values which have been identified as statistically significant, such as raw and supplemental data for the RCP, TNF-$\alpha$, IL-8, and VEGF biomarkers;

$d_{1-n}$ are the discrete variables, such as discrete disease risk factors or designators in the supplemental data, that have been identified as statistically significant,

$m_1$ $m_n$ are coefficients associated with each identified variable, and

b is the y-intercept of the equation.

When the remaining variables are defined and their respective coefficients are selected, the biomarker analysis system 100 establishes the resulting equation as the disease status model (716). The biomarker analysis system 100 may establish multiple disease status models as candidates for further evaluation. The biomarker analysis system 100 may generate composite scores for various subjects in the relevant cohorts by multiplying values for the variables in the disease status model by the coefficient determined during modeling and adding the products along with the intercept value (718). The disease status model may comprise, however, any suitable model or relationship for predicting disease status according to the raw data, supplemental data, and/or subject data.

The biomarker analysis system 100 may utilize the results of the analysis of relationships between the disease state and various raw data, supplemental data, and/or subject data to establish diagnosis criteria for determining disease status using data identified as informative. The biomarker analysis system 100 may establish the diagnosis criteria according to any appropriate process and/or techniques. For example, the biomarker analysis system 100 may identify and/or quantify differences between informative data (and/or combinations of informative data) for the disease-positive cohort, and corresponding informative data (and/or combinations of informative data) for the disease-negative cohort.

The biomarker analysis system 100 may compare the composite scores for the respective cohorts to identify one or more cut points in the composite that may indicate a disease-positive or -negative status, for example, the biomarker analysis system 100 may select and/or retrieve one or more diagnosis cut points and compare the composite scores for the respective cohorts to the diagnosis cut points (722). The diagnosis cut points may be selected according to any suitable criteria, such as according to differences in median and/or cumulative frequency of the composite scores for the respective cohorts. Alternatively, the cut points may be regular intervals across the range of composite scores.

The biomarker analysis system 100 may compare the composite score for each member of a cohort to one or more cut points and record a value indicating the result of the comparison as a composite score cut point designator (724). The composite score cut point designator may comprise any suitable value or indicator, such as the difference between the value and the cut point or other value. If a composite score is above the cut point, an integral value of 1 may be recorded as the composite score cut point designator; if the level is below the cut point, an integral value of 0 is recorded. The integral values could likewise indicate whether the composite scores are below more than one cut point.

To determine the appropriate cut-point for determining disease-positive or disease-negative status, each cohort subject's composite score may be suitably evaluated at different cut-points which span the data's range. At each cut point, values that are equal to or less than the cut point may be considered disease-negative and values above the cut point may be considered disease-positive point, or vice versa according to the nature of the relationship between the data and the disease. The biomarker analysis system 100 may compare the composite score cut point designator for each cut point candidate to each cohort member's true diagnostic state (726), and quantify the test's performance at each cut-point (728), for example as defined by sensitivity, specificity, true positive fraction, true negative Traction, false positive fraction, false negative traction, and so on. From the range of evaluated cut-points, the biomarker analysis system 100 may select one or more cut points for future evaluations of data such that sensitivity is maximized specificity is maximized, or the overall lest performance is maximized as a compromise between maximum sensitivity and specificity.

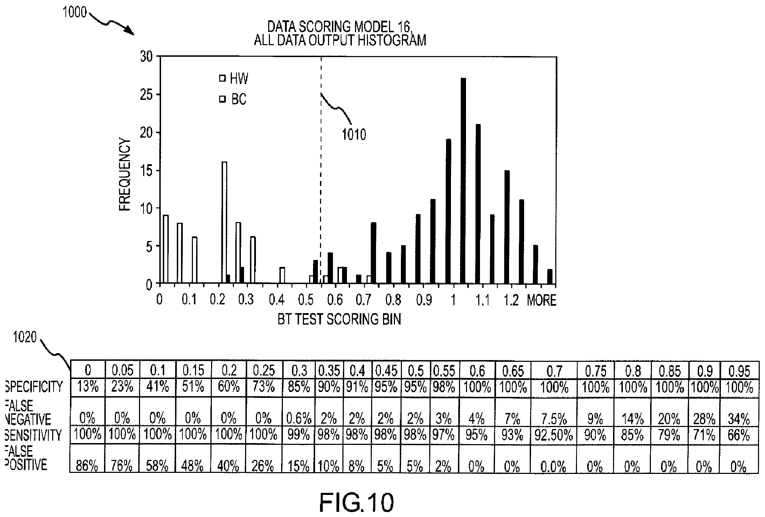

Referring now to Figure 10, an appropriate cut point may be selected by using a data scoring model 1000. In this example, the data scoring model 1000 includes a table 1020 that indicates test accuracy for specificity and sensitivity at various cut points. Using the data provided in the table 1020, the biomarker analysis system 100 may select a cut point 1010 to provide an optimum balance between sensitivity and specificity, such as at .55 in the present example.

The biomarker analysis system 100 may also be configured to verify validity of the disease status model. For example, the biomarker analysis system 100 may receive blind data from disease-negative and disease-positive individuals. The blind data may be analyzed to arrive at diagnoses that may he compared to actual diagnoses to confirm that the disease stale model distinguishes disease-negative and disease-positive solely on the basis of the values of measured and determined variables. If several models are viable, the model that has the highest agreement with the clinical diagnosis may be selected for further evaluation of subjects.

After the disease status model has been established, the biomarker analysis system 100 may analyze biological sample data and/or subject data to apply the disease status model as an indicator of disease status of individual patients. The relevant biomarker levels may be measured and provided to the biomarker analysis system 100, along with relevant subject data.

The biomarker analysis system 100 may process the biomarker data and subject data, for example to adjust the biomarker levels in view of any relevant biomarker factors. The biomarker analysis system 100 may not utilize various variables, such as one or more integral values associated with a biomarker specific cut-point, reduced values, integral values denoting extraordinary values, and raw or normalized data. Data that is not needed for the particular disease status model may be discarded. The biomarker analysis system 100 may use and/or generate only relevant biomarkers and variables, which are those that demonstrate statistical significance and/or are used in the disease status model, to evaluate individual patients. For example, if the disease status model originally considered the PSA, TL-6, RCP, TNF-$\alpha$, IL-8, and VEGF biomarkers, but discarded the PSA and IL-6 biomarkers as insignificant or less significant biomarkers, the biomarker analysis system 100 may discard data for the PSA and IL-6 biomarkers and proceed with analysis of the RCP, TNF-$\alpha$, IL-8, and VEGF biomarkers.

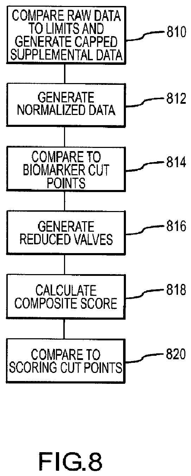

Referring to Figure 8, the biomarker analysis system 100 may perform any suitable processing of the raw biomarker data and other patient information. For example, the biomarker analysis system 100 may establish for each of the patient's relevant biomarker levels a designator, such as an integral value, that indicates whether the level for each biomarker exceeds the relevant biomarker-specific maximum allowable value designated in the disease status model (810). The biomarker analysis system 100 may also associate the corresponding designators with the patient's supplemental data set, indicating that the raw value exceeded the relevant limit.

In addition, the biomarker analysis system 100 may generate normalized data for the patient according to the normalization criteria established in generating the disease status model and the subject data for the patient, such as the patient's age, smoking habits, and the like (812). The normalized data may be added to the supplemental data for the patient.

The biomarker analysis system 100 may also compare the patient's raw data and/or supplemental data to the biomarker cut points and generate cut point designators for each relevant biomarker cut point and the corresponding data (814). The biomarker analysis system 100 may further establish reduced data values for the each of the patient's relevant measured biomarker levels, for example by raising the relevant data to the fractional powers used by the disease status model, and associating alt such reduced data values with the patient's data set (816).

The biomarker analysis system 100 may evaluate the raw biomarker data and any other relevant data in conjunction with the disease status model. For example, the biomarker analysis system 100 may calculate a composite score for the patient using the patient's biomarker data and other data and the disease status model (818). The biomarker analysis system 100 may compare the composite score to the scoring model cut points (820).

Scores above the cut point suggest that the disease status of the subject is positive, while scores below the cut point indicate that the subject is negative. The biomarker analysis system 100 may also compare the composite score to boundary definitions for indeterminate zone that may be constructed around the cut-point where no determination can be made. The indeterminate zone may account, for example, for both a patient's biological variability (the typical day to day variations in the biomarkers of interest) and the evaluation methods error.

**Claims**

**1.** A method for assessing a breast cancer disease status of a patient, comprising:

obtaining a first data set for a plurality of biomarkers in a condition-positive cohort;
obtaining a second data set for the plurality of biomarkers in a condition-negative cohort; wherein the plurality of biomarkers comprises prostate-specific antigen (PSA), tumor necrosis factor alpha (TNF-$\alpha$), interleukin-6 (IL-6), interleukin-8 (IL-8), vascular endothelial growth factor (VEGF), and riboflavin carrier protein (RCP);
processing the first data set and the second data set to reduce the influence of non-normal biomarker levels with a non-Gaussian distribution within the condition-positive cohort and the condition-negative cohort by assigning maximum and/or minimum allowable values for each biomarker to produce a first processed data set and a second processed data set;
generating a disease status model by selection of at least one informative biomarker from the first processed data set as compared to the second processed data set using an analysis configured to remove a biomarker that is uninformative of disease status;
inputting a patient data set for the at least one informative biomarker into the disease status model; and
determining the breast cancer disease status of the patient.

2. The method of claim 1, wherein the first processed data set and the second processed data set are generated by reducing the range in value of the first data set and the second data set and the disease status model is generated by comparing the first processed data set to the second processed data set; and wherein generating the first processed data set and the second processed data set comprises raising the first data set and the second data set by a fractional exponent.

3. The method of claim 1, wherein the first processed data set and the second processed data set are produced by comparing a cumulative frequency distribution of each biomarker in the first data set with a cumulative frequency distribution of the corresponding biomarker in the second data set and selecting a cut point for each biomarker using a maximum difference between the cumulative frequency distribution of each biomarker in the first data set and the cumulative frequency distribution for the corresponding biomarker in the second data set,
further comprising:

comparing the first processed data set and the second processed data set to the cut point; and
generating a cut point data set comprising a set of discrete values by determining whether each datum compared to the cut point exceeded the cut point.

4. The method of claim 1, wherein the processing of the first data set and the second data set comprises:

comparing the first data set and the second data set to a threshold;
generating multiple discrete values for the first data set and the second data set compared to the threshold as a result of the comparison; and
generating the disease status model for determining the disease status by determining differences between the discrete values for the at least one informative biomarker of the first data set and the discrete values for the at least one informative biomarker of the second data set.

5. The method of claim 4, wherein the processing of the first data set and the second data set further comprises: generating capped biomarker data for the first data set and second data set, wherein capped biomarker data comprises:

the first data set and the second data set for data within a cap limit; and
a cap value for the first data set and the second data set that exceeds the cap limit.

6. The method of claim 5, further comprising selecting the cap limit from a median value of at least one of the first data set and the second data set.

7. The method of claim 1, wherein:

the disease status model comprises at least one dependent variable and at least one independent variable,
the disease status is a dependent variable; and
the at least one informative biomarker comprises the at least one independent variable.

8. A system for assessing a breast cancer disease status of a patient, comprising a computer system configured to:

receive a first data set for a plurality of biomarkers in a condition-positive cohort;
receive a second data set for a plurality of biomarkers in a condition-negative cohort;
wherein the plurality of biomarkers comprises prostate-specific antigen (PSA), tumor necrosis factor alpha (TNF-$\alpha$), interleukin-6 (IL-6), interleukin-8 (IL-8), vascular endothelial growth factor (VEGF), and riboflavin carrier protein (RCP); process the first data set and the second data set to reduce the influence of non-normal biomarker levels with a non-Gaussian distribution within the condition-positive cohort and the condition-negative cohort by assigning maximum and/or minimum allowable values for each biomarker to produce a first processed data set and a second processed data set; and
generate a disease status model by selection of at least one informative biomarker from the first processed data set as compared to the second processed data set using an analysis configured to remove a biomarker that is uninformative of disease status;

inputting a patient data set for the at least one informative biomarker into the disease status model; and determining the breast cancer disease status of the patient.

9. The system of claim 8, wherein the computer system is further configured to:

generate a reduced range in value first data set and a reduced range in value second data set; and automatically generate a disease status model using a difference between the first processed data set and the second processed data set; and

wherein the computer system is configured to generate the first processed data set and the second processed data set by raising the first data set and the second data set by a fractional exponent.

10. The system of claim 8, wherein the computer system is further configured to:

compare a cumulative frequency distribution of each biomarker in the first data set with a cumulative frequency distribution of the corresponding biomarker in the second data set;

select a cut point for each biomarker using a maximum difference between the cumulative frequency distribution of each biomarker in the first data set and the cumulative frequency distribution for the corresponding biomarker in the second data set.

11. The system of claim 10, wherein the computer system is further configured to:

compare the first data set and the second data set to the cut point; and

generate a cut point data set comprising a set of discrete values by determining whether each datum compared to the cut point exceeded the cut point.

12. The system of claim 8, wherein the computer system is further configured to:

compare the first data set and the second data set to a threshold;

generate multiple discrete values for the first data set and the second data set compared to the threshold as a result of the comparison; and

automatically generate the disease status model for determining the disease status by determining differences between the discrete values for the at least one informative biomarker of the first data set and the discrete values for the at least one informative biomarker of the second data set.

13. The system of claim 12, wherein the computer system is further configured to generate capped biomarker data for the first data set and the second data set, wherein capped biomarker data comprises:

the first data set and the second data set for data within a cap limit; and

a cap value for the first data set and the second data set that exceeds the cap limit.

14. The system of claim 13, wherein the computer system is further configured to select the cap limit from a median value of at least one of the first data set and the second data set.

15. The system of claim 8, wherein:

the disease status model comprises at least one dependent variable and at least one independent variable, the disease status is a dependent variable; and

the at least one informative biomarker comprises the at least one independent variable.

16. A computer program configured to cause a computer to execute a method for assessing a disease status of a human according to the method of claim 1.

**Patentansprüche**

1. Verfahren zum Beurteilen des Brustkrebserkrankungsstatus eines Patienten, das Folgendes beinhaltet:

Einholen eines ersten Datensatzes für eine Mehrzahl von Biomarkern in einer zustandspositiven Kohorte;
Einholen eines zweiten Datensatzes für die Mehrzahl von Biomarkern in einer zustandsnegativen Kohorte;
wobei die Mehrzahl von Biomarkern prostataspezifisches Antigen (PSA), Tumornekrosefaktor alpha (TNF-$\alpha$), Interleukin-6 (IL-6), Interleukin-8 (IL-8), Vascular Endothelial Growth Factor (VEGF) und Riboflavinträgerprotein (RCP) umfasst;
Verarbeiten des ersten Datensatzes und des zweiten Datensatzes zum Reduzieren des Einflusses von nicht normalen Biomarkerspiegeln mit einer nichtgaußförmigen Verteilung in der zustandspositiven Kohorte und der zustandsnegativen Kohorte durch Zuordnen von maximalen und/oder minimalen zulässigen Werten für jeden Biomarker, um einen ersten verarbeiteten Datensatz und einen zweiten verarbeiteten Datensatz zu erzeugen;
Erzeugen eines Erkrankungsstatusmodells durch Auswählen wenigstens eines informativen Biomarkers aus dem ersten verarbeiteten Datensatz im Vergleich zum zweiten verarbeiteten Datensatz anhand einer Analyse, die zum Entfernen eines Biomarkers konfiguriert ist, der nicht über den Erkrankungsstatus informativ ist;
Eingeben eines Patientendatensatzes für den wenigstens einen informativen Biomarker in das Erkrankungsstatusmodell; und
Bestimmen des Brustkrebserkrankungsstatus des Patienten.

2. Verfahren nach Anspruch 1, wobei der erste verarbeitete Datensatz und der zweite verarbeitete Datensatz durch Reduzieren des Wertebereichs des ersten Datensatzes und des zweiten Datensatzes erzeugt werden und das Erkrankungsstatusmodell durch Vergleichen des ersten verarbeiteten Datensatzes mit dem zweiten verarbeiteten Datensatz erzeugt wird; und wobei das Erzeugen des ersten verarbeiteten Datensatzes und des zweiten verarbeiteten Datensatzes das Erheben des ersten Datensatzes und des zweiten Datensatzes um einen Bruchexponenten beinhaltet.

3. Verfahren nach Anspruch 1, wobei der erste verarbeitete Datensatz und der zweite verarbeitete Datensatz durch Vergleichen einer kumulativen Frequenzverteilung jedes Biomarkers im ersten Datensatz mit einer kumulativen Frequenzverteilung des entsprechenden Biomarkers im zweiten Datensatz und das Auswählen eines Trennpunkts für jeden Biomarker mit einer maximalen Differenz zwischen der kumulativen Frequenzverteilung jedes Biomarkers im ersten Datensatz und der kumulativen Frequenzverteilung des entsprechenden Biomarkers im zweiten Datensatz beinhaltet,
das ferner Folgendes beinhaltet:

Vergleichen des ersten verarbeiteten Datensatzes und des zweiten verarbeiteten Datensatzes mit dem Trennpunkt; und
Erzeugen eines Trennpunktdatensatzes, der einen Satz von diskreten Werten umfasst, durch Feststellen, ob jeder Bezugspunkt im Vergleich zum Trennpunkt den Trennpunkt überschritten hat.

4. Verfahren nach Anspruch 1, wobei das Verarbeiten des ersten Datensatzes und des zweiten Datensatzes Folgendes beinhaltet:

Vergleichen des ersten Datensatzes und des zweiten Datensatzes mit einer Schwelle;
Erzeugen mehrerer diskreter Werte für den ersten Datensatz und den zweiten Datensatz im Vergleich zu der Schwelle als ein Ergebnis des Vergleichs; und
Erzeugen des Erkrankungsstatusmodells zum Ermitteln des Erkrankungsstatus durch Feststellen von Differenzen zwischen den diskreten Werten für den wenigstens einen informativen Biomarker des ersten Datensatzes und den diskreten Werten für den wenigstens einen informativen Biomarker des zweiten Datensatzes.

5. Verfahren nach Anspruch 4, wobei das Verarbeiten des ersten Datensatzes und des zweiten Datensatzes ferner Folgendes beinhaltet: Erzeugen von gekappten Biomarkerdaten für den ersten Datensatz und den zweiten Datensatz, wobei die gekappten Biomarkerdaten Folgendes umfassen:

den ersten Datensatz und den zweiten Datensatz für Daten innerhalb einer Kappengrenze; und
einen Kappenwert für den ersten Datensatz und den zweiten Datensatz, der die Kappengrenze übersteigt.

6. Verfahren nach Anspruch 5, das ferner das Auswählen der Kappengrenze aus einem Medianwert des ersten Datensatzes und/oder des zweiten Datensatzes beinhaltet.

7. Verfahren nach Anspruch 1, wobei:

das Erkrankungsstatusmodell wenigstens eine abhängige Variable und wenigstens eine unabhängige Variable umfasst,

der Erkrankungsstatus eine abhängige Variable ist; und

der wenigstens eine informative Biomarker die wenigstens eine unabhängige Variable umfasst.

**8.** System zum Beurteilen eines Brustkrebserkrankungsstatus eines Patienten, das ein Computersystem umfasst, das konfiguriert ist zum:

Empfangen eines ersten Datensatzes für eine Mehrzahl von Biomarkern in einer zustandspositiven Kohorte;

Empfangen eines zweiten Datensatzes für eine Mehrzahl von Biomarkern in einer zustandsnegativen Kohorte;

wobei die Mehrzahl von Biomarkern prostataspezifisches Antigen (PSA), Tumornekrosefaktor alpha (TNF-$\alpha$), Interleukin-6 (IL-6), Interleukin-8 (IL-8), Vascular Endothelial Growth Factor (VEGF) und Riboflavinträgerprotein (RCP) umfasst;

Verarbeiten des ersten Datensatzes und des zweiten Datensatzes zum Reduzieren des Einflusses von nicht normalen Biomarkerspiegeln mit einer nichtgaußförmigen Verteilung in der zustandspositiven Kohorte und der zustandsnegativen Kohorte durch Zuordnen von maximalen und/oder minimalen zulässigen Werten für jeden Biomarker, um einen ersten verarbeiteten Datensatz und einen zweiten verarbeiteten Datensatz zu erzeugen;

Erzeugen eines Erkrankungsstatusmodells durch Auswählen wenigstens eines informativen Biomarkers aus dem ersten verarbeiteten Datensatz im Vergleich zum zweiten verarbeiteten Datensatz anhand einer Analyse, die zum Entfernen eines Biomarkers konfiguriert ist, der nicht über den Erkrankungsstatus informativ ist;

Eingeben eines Patientendatensatzes für den wenigstens einen informativen Biomarker in das Erkrankungsstatusmodell; und

Bestimmen des Brustkrebserkrankungsstatus des Patienten.

**9.** System nach Anspruch 8, wobei das Computersystem ferner konfiguriert ist zum:

Erzeugen eines ersten Datensatzes mit reduziertem Wertebereich und eines zweiten Datensatzes mit reduziertem Wertebereich; und

automatisches Erzeugen eines Erkrankungsstatusmodells anhand einer Differenz zwischen dem ersten verarbeiteten Datensatz und dem zweiten verarbeiteten Datensatz; und

wobei das Computersystem zum Erzeugen des ersten verarbeiteten Datensatzes und des zweiten verarbeiteten Datensatzes durch Erheben des ersten Datensatzes und des zweiten Datensatzes um einen Bruchexponenten konfiguriert ist.

**10.** System nach Anspruch 8, wobei das Computersystem ferner konfiguriert ist zum:

Vergleichen einer kumulativen Frequenzverteilung jedes Biomarkers im ersten Datensatz mit einer kumulativen Frequenzverteilung des entsprechenden Biomarkers im zweiten Datensatz;

Auswählen eines Trennpunkts für jeden Biomarker anhand einer maximalen Differenz zwischen der kumulativen Frequenzverteilung jedes Biomarkers in dem ersten Datensatz und der kumulativen Frequenzverteilung für den entsprechenden Biomarker im zweiten Datensatz.

**11.** System nach Anspruch 10, wobei das Computersystem ferner konfiguriert ist zum:

Vergleichen des ersten Datensatzes und des zweiten Datensatzes mit dem Trennpunkt; und

Erzeugen eines Trennpunktdatensatzes, der einen Satz von diskreten Werten umfasst, durch Bestimmen, ob jeder Bezugspunkt im Vergleich zum Trennpunkt den Trennpunkt überschritten hat.

**12.** System nach Anspruch 8, wobei das Computersystem ferner konfiguriert ist zum:

Vergleichen des ersten Datensatzes und des zweiten Datensatzes mit einer Schwelle;

Erzeugen mehrerer diskreter Werte für den ersten Datensatz und den zweiten Datensatz im Vergleich zu der Schwelle als Ergebnis des Vergleichs; und

automatischen Erzeugen des Erkrankungsstatusmodells zum Bestimmen des Erkrankungsstatus durch Bestimmen von Differenzen zwischen den diskreten Werten für den wenigstens einen informativen Biomarker des ersten Datensatzes und den diskreten Werten für den wenigstens einen informativen Biomarker des zweiten Datensatzes.

**13.** System nach Anspruch 12, wobei das Computersystem ferner konfiguriert ist zum:

Erzeugen von gekappten Biomarkerdaten für den ersten Datensatz und den zweiten Datensatz, wobei gekappte Biomarkerdaten Folgendes umfassen:

den ersten Datensatz und den zweiten Datensatz für Daten innerhalb einer Kappengrenze; und
einen Kappenwert für den ersten Datensatz und den zweiten Datensatz, der die Kappengrenze übersteigt.

**14.** System nach Anspruch 13, wobei das Computersystem ferner zum Auswählen der Kappengrenze von einem Medianwert von wenigstens einem aus dem ersten Datensatz und dem zweiten Datensatz konfiguriert ist.

**15.** System nach Anspruch 8, wobei:

das Erkrankungsstatusmodell wenigstens eine abhängige Variable und wenigstens eine unabhängige Variable umfasst,
der Erkrankungsstatus eine abhängige Variable ist; und
der wenigstens eine informative Biomarker die wenigstens eine unabhängige Variable umfasst.

**16.** Computerprogramm, konfiguriert zum Bewirken, dass ein Computer ein Verfahren zum Beurteilen eines Erkrankungsstatus eines Menschen gemäß dem Verfahren nach Anspruch 1 ausführt.

**Revendications**

**1.** Procédé pour identifier un état pathologique de cancer du sein d'un patient, comprenant :

obtenir un premier ensemble de données pour une pluralité de biomarqueurs dans une cohorte positive pour la condition ;
obtenir un deuxième ensemble de données pour la pluralité de biomarqueurs dans une cohorte négative pour la condition ; dans lequel la pluralité de biomarqueurs comprend l'antigène spécifique de la prostate (PSA), le facteur de nécrose tumorale alpha (TNF-$\alpha$), l'interleukine-6 (IL-6), l'interleukine-8 (IL-8), le facteur de croissance de l'endothélium vasculaire (VEGF), et la protéine porteuse de riboflavine (RCP) ;
traiter le premier ensemble de données et le deuxième ensemble de données pour réduire l'influence de taux de biomarqueurs non normaux avec une distribution non gaussienne dans la cohorte positive pour la condition et dans la cohorte négative pour la condition en attribuant des valeurs admissibles maximales et/ou minimales pour chaque biomarqueur afin de produire un premier ensemble de données traité et un deuxième ensemble de données traité ;
générer un modèle d'état pathologique en sélectionnant au moins un biomarqueur informatif du premier ensemble de données traité tel que comparé au deuxième ensemble de données traité en utilisant une analyse configurée pour retirer un biomarqueur qui n'est pas informatif de l'état pathologique ;
entrer un ensemble de données d'un patient pour le au moins un biomarqueur informatif dans le modèle d'état pathologique ; et
déterminer l'état pathologique de cancer du sein du patient.

**2.** Procédé selon la revendication 1, dans lequel le premier ensemble de données traité et le deuxième ensemble de données traité sont générés en réduisant la plage dans la valeur du premier ensemble de données et du deuxième ensemble de données et le modèle d'état pathologique est généré en comparant le premier ensemble de données traité au deuxième ensemble de données traité ; et dans lequel générer le premier ensemble de données traité et le deuxième ensemble de données traité comprend élever le premier ensemble de données et le deuxième ensemble de données par un exposant fractionnaire.

**3.** Procédé selon la revendication 1, dans lequel le premier ensemble de données traité et le deuxième ensemble de données traité sont produits en comparant une distribution de fréquence cumulative de chaque biomarqueur dans le premier ensemble de données à une distribution de fréquence cumulative du biomarqueur correspondant dans le deuxième ensemble de données et en sélectionnant un point de coupure pour chaque biomarqueur en utilisant une différence maximale entre la distribution de fréquence cumulative de chaque biomarqueur dans le premier ensemble de données et la distribution de fréquence cumulative pour le biomarqueur correspondant dans le deuxième ensemble de données,

comprenant en outre :

comparer le premier ensemble de données traité et le deuxième ensemble de données traité au point de coupure ; et

générer un ensemble de données de point de coupure comprenant un ensemble de valeurs discrètes en déterminant si chaque donnée comparée au point de coupure a dépassé le point de coupure.

**4.** Procédé selon la revendication 1, dans lequel le traitement du premier ensemble de données et du deuxième ensemble de données comprend :

comparer le premier ensemble de données et le deuxième ensemble de données à un seuil ;

générer de multiples valeurs discrètes pour le premier ensemble de données et le deuxième ensemble de données comparés au seuil comme un résultat de la comparaison ; et

générer le modèle d'état pathologique pour déterminer l'état pathologique en déterminant des différences entre les valeurs discrètes pour le au moins un biomarqueur informatif du premier ensemble de données et les valeurs discrètes pour le au moins un biomarqueur informatif du deuxième ensemble de données.

**5.** Procédé selon la revendication 4, dans lequel le traitement du premier ensemble de données et du deuxième ensemble de données comprend en outre : générer des données de biomarqueur plafonnées pour le premier ensemble de données et le deuxième ensemble de données, dans lequel les données de biomarqueur plafonnées comprennent :

le premier ensemble de données et le deuxième ensemble de données pour des données dans une limite de plafond ; et

une valeur de plafond pour le premier ensemble de données et le deuxième ensemble de données qui dépasse la limite de plafond.

**6.** Procédé selon la revendication 5, comprenant en outre sélectionner la limite de plafond parmi une valeur médiane d'au moins l'un d'entre le premier ensemble de données et le deuxième ensemble de données.

**7.** Procédé selon la revendication 1, dans lequel :

le modèle d'état pathologique comprend au moins une variable dépendante et au moins une variable indépendante,

l'état pathologique est une variable dépendante ; et

le au moins un biomarqueur informatif comprend la au moins une variable indépendante.

**8.** Système d'évaluation d'un état pathologique de cancer du sein d'un patient, comprenant un système informatique configuré pour :

recevoir un premier ensemble de données pour une pluralité de biomarqueurs dans une cohorte positive pour la condition ;

recevoir un deuxième ensemble de données pour une pluralité de biomarqueurs dans une cohorte négative pour la condition ;

dans lequel la pluralité de biomarqueurs comprend l'antigène spécifique de la prostate (PSA), le facteur de nécrose tumorale alpha (TNF-$\alpha$), l'interleukine-6 (IL-6), finterleukine-8 (IL-8), le facteur de croissance de l'endothélium vasculaire (VEGF), et la protéine porteuse de riboflavine (RCP) ; traiter le premier ensemble de données et le deuxième ensemble de données pour réduire l'influence de taux de biomarqueurs non normaux avec une distribution non gaussienne dans la cohorte positive pour la condition et dans la cohorte négative pour la condition en attribuant des valeurs admissibles maximales et/ou minimales pour chaque biomarqueur afin de produire un premier ensemble de données traité et un deuxième ensemble de données traité ;

générer un modèle d'état pathologique en sélectionnant au moins un biomarqueur informatif du premier ensemble de données traité tel que comparé au deuxième ensemble de données traité en utilisant une analyse configurée pour retirer un biomarqueur qui n'est pas informatif de l'état pathologique ;

entrer un ensemble de données d'un patient pour le au moins un biomarqueur informatif dans le modèle d'état pathologique ; et

déterminer l'état pathologique de cancer du sein du patient.

**9.** Système selon la revendication 8, dans lequel le système informatique est configuré en outre pour :

générer une plage réduite dans la valeur du premier ensemble de données et une plage réduite dans la valeur du deuxième ensemble de données ; et

générer automatiquement un modèle d'état pathologique en utilisant une différence entre le premier ensemble de données traité et le deuxième ensemble de données traité ; et

dans lequel le système informatique est configuré pour générer le premier ensemble de données traité et le deuxième ensemble de données traité en élevant le premier ensemble de données et le deuxième ensemble de données par un exposant fractionnaire.

**10.** Système selon la revendication 8, dans lequel le système informatique est configuré en outre pour :

comparer une distribution de fréquence cumulative de chaque biomarqueur dans le premier ensemble de données à une distribution de fréquence cumulative du biomarqueur correspondant dans le deuxième ensemble de données ;

sélectionner un point de coupure pour chaque biomarqueur en utilisant une différence maximale entre la distribution de fréquence cumulative de chaque biomarqueur dans le premier ensemble de données et la distribution de fréquence cumulative pour le biomarqueur correspondant dans le deuxième ensemble de données.

**11.** Système selon la revendication 10, dans lequel le système informatique est configuré en outre pour :

comparer le premier ensemble de données et le deuxième ensemble de données au point de coupure ; et

générer un ensemble de données de point de coupure comprenant un ensemble de valeurs discrètes en déterminant si chaque donnée comparée au point de coupure a dépassé le point de coupure.

**12.** Système selon la revendication 8, dans lequel le système informatique est configuré en outre pour :

comparer le premier ensemble de données et le deuxième ensemble de données à un seuil ;

générer de multiples valeurs discrètes pour le premier ensemble de données et le deuxième ensemble de données comparés au seuil comme un résultat de la comparaison ; et

générer automatiquement le modèle d'état pathologique pour déterminer l'état pathologique en déterminant des différences entre les valeurs discrètes pour le au moins un biomarqueur informatif du premier ensemble de données et les valeurs discrètes pour le au moins un biomarqueur informatif du deuxième ensemble de données.

**13.** Système selon la revendication 12, dans lequel le système informatique est configuré en outre pour :

générer des données de biomarqueur plafonnées pour le premier ensemble de données et le deuxième ensemble de données, dans lequel les données de biomarqueur plafonnées comprennent :

le premier ensemble de données et le deuxième ensemble de données pour des données dans une limite de plafond ; et

une valeur de plafond pour le premier ensemble de données et le deuxième ensemble de données qui dépasse la limite de plafond.

**14.** Système selon la revendication 13, dans lequel le système informatique est configuré en outre pour sélectionner la limite de plafond parmi une valeur médiane d'au moins l'un d'entre le premier ensemble de données et le deuxième ensemble de données.

**15.** Système selon la revendication 8, dans lequel :

le modèle d'état pathologique comprend au moins une variable dépendante et au moins une variable indépendante,

l'état pathologique est une variable dépendante ; et

le au moins un biomarqueur informatif comprend la au moins une variable indépendante.

**16.** Programme informatique configuré pour faire qu'un ordinateur exécute un procédé pour évaluer un état pathologique d'un être humain conformément au procédé selon la revendication 1.

FIG.1

FIG.2

FIG.3

ANALYZE BIOMARKER
FACTORS FOR
SIGNIFICANCE — 410

GENERATE EQUATION
RELATING SUBJECT
DATA TO RAW DATA — 412

INVERT
EQUATION — 414

GENERATE
NORMALIZED DATA — 416

# FIG.4

```
┌─────────────────┐
│ PLOT CUMULATIVE │──── 510
│    FREQUENCY    │
│  DISTRIBUTIONS  │
└─────────────────┘
         │
┌─────────────────┐
│     SELECT      │──── 512
│    CUT POINT    │
└─────────────────┘
         │
┌─────────────────┐
│  COMPARE DATA   │──── 514
│   TO CUT POINT  │
└─────────────────┘
         │
┌─────────────────┐
│    GENERATE     │──── 516
│    CUT POINT    │
│   DESIGNATORS   │
└─────────────────┘
```

# FIG.5

CUMULATIVE FREQUENCY TO ELIMINATE MARKER CONFUSION

FIG.6

FIG.7

COMPARE RAW DATA TO LIMITS AND GENERATE CAPPED SUPPLEMENTAL DATA — 810

GENERATE NORMALIZED DATA — 812

COMPARE TO BIOMARKER CUT POINTS — 814

GENERATE REDUCED VALVES — 816

CALCULATE COMPOSITE SCORE — 818

COMPARE TO SCORING CUT POINTS — 820

FIG.8

EP 2 016 405 B1

FIG.9

1000

DATA SCORING MODEL 16,
ALL DATA OUTPUT HISTOGRAM

□ HW

□ BC

1010

FREQUENCY

BT TEST SCORING BIN

1020

| | 0 | 0.05 | 0.1 | 0.15 | 0.2 | 0.25 | 0.3 | 0.35 | 0.4 | 0.45 | 0.5 | 0.55 | 0.6 | 0.65 | 0.7 | 0.75 | 0.8 | 0.85 | 0.9 | 0.95 |
|---|---|---|---|---|---|---|---|---|---|---|---|---|---|---|---|---|---|---|---|---|
| SPECIFICITY | 13% | 23% | 41% | 51% | 60% | 73% | 85% | 90% | 91% | 95% | 95% | 98% | 100% | 100% | 100% | 100% | 100% | 100% | 100% | 100% |
| FALSE NEGATIVE | 0% | 0% | 0% | 0% | 0% | 0% | 0.6% | 2% | 2% | 2% | 2% | 3% | 4% | 7% | 7.5% | 9% | 14% | 20% | 28% | 34% |
| SENSITIVITY | 100% | 100% | 100% | 100% | 100% | 100% | 99% | 98% | 98% | 98% | 98% | 97% | 95% | 93% | 92.50% | 90% | 85% | 79% | 71% | 66% |
| FALSE POSITIVE | 86% | 76% | 58% | 48% | 40% | 26% | 15% | 10% | 8% | 5% | 5% | 2% | 0% | 0% | 0.0% | 0% | 0% | 0% | 0% | 0% |

FIG.10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6714925 B
- US 2005176057 A

- US 6949342 B

**Non-patent literature cited in the description**

- Molecular classification of cancer: Class discovery and class prediction by gene expression monitoring. **GOLUB T R et al.** Science. American Association for the Advancement of Science, vol. 286, 531-537